# EUROPEAN PATENT APPLICATION

(11) **EP 1 166 742 A2**
(43) Date of publication of application: **02.01.2002**
(21) Application number: 01113642.1
(22) Date of filing: 18.06.2001
(51) Int. Cl.: A61J 1/20

(54) **Kit for medicament reconstitution including a prefilled syringe**

(30) Priority: 22.06.2000 JP 2000187449
(71) Applicant: Nipro Corporation, Kita-ku, Osaka-shi, Osaka-fu, 531-8510 (JP); TORII PHARMACEUTICAL CO., LTD., Chuo-ku, Tokyo 103 (JP)
(72) Inventor: Yagi, Hideki, Kita-ku, Osaka-shi, Osaka-fu (JP); Hama, Yoshihisa, Kita-ku, Osaka-shi, Osaka-fu (JP); Aramata, Masafumi, Kita-ku, Osaka-shi, Osaka-fu (JP); Natsui, Kensuke, Torii Pharmaceutical Co., Ltd., Chuo-ku, Tokyo-to (JP)
(74) Representative: VOSSIUS & PARTNER

(57) **Abstract**

A solution kit includes a prefilled syringe (1) and a container (2) containing a solvent charged therein. The prefilled syringe comprises a barrel (11) provided at a distal end thereof with a tip (111) closed by a twist-off type closing member (112), while the solvent container having a mouth portion (22) closed by a twist-off type closing member (23). The tip of the prefilled syringe and the mouth portion of the solvent container are configured to engage with one another in liquid-tight engagement.

## Description

The present invention relates to a solution kit and, more particularly, to a solution kit comprising a syringe containing a freeze-dried medicine and a solvent container containing a solvent, which makes it possible to efficiently perform freeze-drying of an injectable medicine and aseptic and simple dissolution of the freeze-dried medicine in the solvent before use.

Up to now, prefilled syringes that have been previously filled with an injectable medicine, have been used, in order to prevent misuse, misoperation, contamination with foreign substances, accidental or erroneous puncture or the like which may occur during transfer of a liquid medicine from a vial or medicine container into a syringe. However, the most injectable medicines in a liquid state rapidly change in quality and lose their validity. Cold storage or special packaging would extend the shelf life of the liquid medicines, but these processes result in increase of production cost and complication of the storage.

In recent years, it has been proposed to use a prefilled syringe of the kind wherein a liquid medicine charged in a syringe is freeze-dried to prevent the change in quality during storage (e.g., JP-A-H07-75673, JP-A-H07-80064 and JP-A-H07-213609). The prefilled syringe of this type makes it possible to subcutaneously inject a dose of the medicine by mixing the freeze-dried medicine charged in the syringe with a solvent just before use.

The prefilled syringes disclosed in JP-A-H07-75673 and JP-A-H07-80064 are of a two-compartment type in which an interior of the syringe is partitioned by an intermediate stopper and a liquid chamber stopper. The syringe of JP-A-H07-75763 is provided at a proximal portion of its barrel with a vent bypass that extends from a proximal end of the barrel toward the distal end thereof and has a length shorter than a thickness of the intermediate stopper. This vent bypass allows an interior of the syringe to be communicated with the atmosphere therethrough when an approximately half of the intermediate stopper is put in the barrel.

On the other hand, the syringe of JP-A-H07-80064 is provided in a proximal portion of a barrel with a vent hole at a position spaced from the proximal end of the barrel by a distance smaller than the thickness of the intermediate stopper. The vent hole allows an interior of the syringe to be communicated with the atmosphere therethrough when an approximately half of the intermediate stopper is put in the barrel.

The prefilled syringe of JP-A-H07-213609 comprises a barrel and a plunger stopper, said plunger stopper having a distal portion provided with a vapor passage and a proximal portion configured for sliding fluid-tight engagement with the barrel. The vapor passage extends from the distal end of the barrel toward the proximal end thereof to allow an interior of the barrel to be communicated with the atmosphere therethrough when an approximately half of the intermediate stopper is put in the barrel.

However, these two-compartment type syringes are restricted in usable solvent and require an aseptic room for charging the syringe with the medicine and solvent, which increases cost disadvantages. In addition, the prefilled syringe with the specially configured plunger stopper is increased in size correspondingly to the lengthened plunger stopper. This causes increase in consumption of a relatively expensive rubber, resulting in increase of cost disadvantages.

The present invention has been made in view of the above circumstances to provide a solution kit including a syringe prefilled with a freeze-dried medicine, which is easy to perform operation to dissolve the freeze-dried medicine in a solvent and increases cost advantages.

According to the present inventors, the above object is achieved by a combination of a prefilled syringe and a solvent container, wherein a needle-receiving portion (tip) at a distal end of the prefilled syringe and a mouth portion of the solvent container are configured to permit fluid-tight engagement between them.

According to the present invention, there is provided a solution kit comprising:
a prefilled syringe comprising a barrel provided at a distal end thereof with a tip, a freeze-dried medicine prefilled in said barrel, and a gasket arranged in said barrel through a proximal end thereof, said tip being closed by a twist-off type closing member; and
a solvent container having a mouth portion closed by a twist-off type closing member, said container containing a solvent charged therein,
said tip of the prefilled syringe and the mouth portion of the solvent container being configured to engage with one another in liquid-tight engagement.

The solution kit may include an additional member for twisting off the closure member. In this case, the closure member on the tip of the barrel is formed into a prismatic form and the additional member is provided with a recess portion with a configuration complementary to the outer shape of the tip and closure member integrally formed therewith. Further, the tip is preferably configured for insertion into or engagement with the mouth portion of the solvent container.

The present invention will become more fully understood from the detailed description given below and the accompanying drawings, which are given by way of illustration only and thus are not limitative of the present invention.
Fig. 1 is a cross-sectional view of one embodiment of a solution kit according to the present invention;
Fig. 2 is an enlarged cross-sectional view of a prefilled syringe used in the kit of Fig. 1 with an additional member for twisting off the closure member attached to a tip of the syringe;

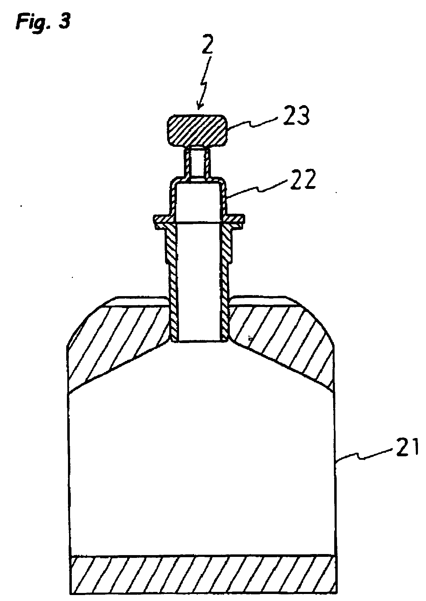

Fig. 3 is an enlarged cross-sectional view of a solvent container used in the kit of Fig. 1.
Fig. 4 is a cross section illustrating an assembled state of the solution kit including the prefilled syringe of Fig. 1 and the solvent container of Fig. 1.

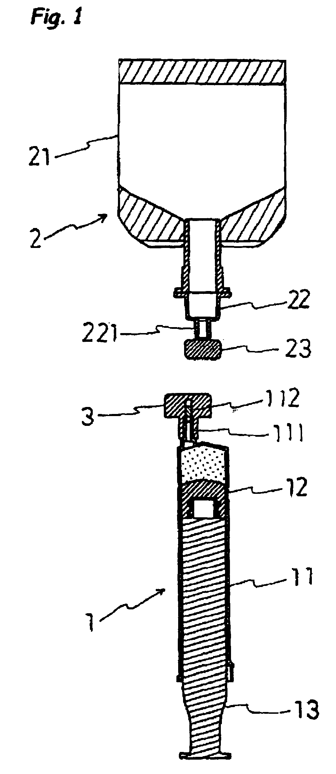

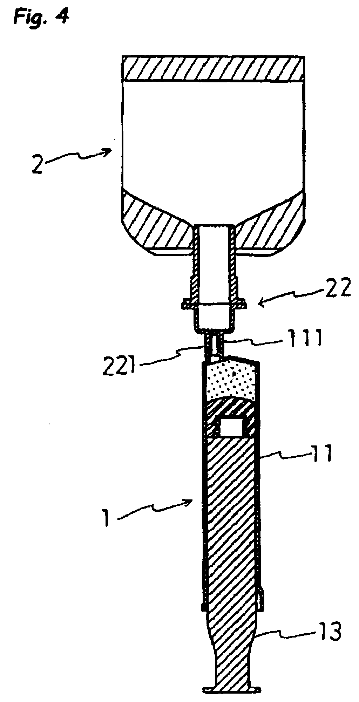

As shown in Fig. 1, the solution kit of the present invention comprises a prefilled syringe 1 containing a freeze-dried medicine (not shown in the drawing), and a solvent container 2 containing a solvent (not shown in the drawing) charged therein. The prefilled syringe 1 comprises a barrel 11 having a tip 111 at a distal end thereof, which is closed by a twist-off type closing member 112, and a gasket 12 arranged in the barrel 11 through a proximal end thereof. The solvent container 2 is provided at its distal end with a mouth portion 22, which is closed by a twist-off type closing member 23. The tip 111 of the prefilled syringe 1 and the mouth portion 22 of the solvent container 2 are configured to engage with each other in fluid-tight engagement, as shown in Fig. 4.

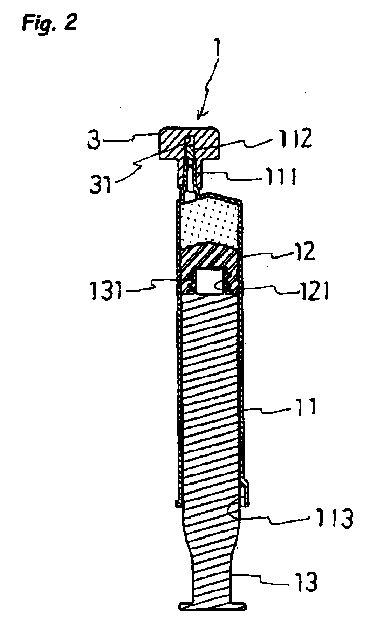

As shown in Fig. 2, the prefilled syringe 1 comprises the barrel 11 provided at a distal end thereof with a tip 111 for attachment of a needle, a freeze-dried medicine (not shown in the drawing) contained therein, and a gasket 12 inserted into the barrel 11 through the proximal end thereof and disposed at an intermediate portion of the barrel 11. The barrel 11 is made of a resin such as polyethylene or polypropylene. The twist-off type closing member 112 for closing the tip 111 of the barrel 11 is made of the same material as that of the barrel 11 and integrally formed with the tip 111.

It is preferred to provide the tip 111 and the closure member 112 with an additional member 3 for twisting off the closure member 112. The additional member 3 for twisting off the closure member 112 is a cap-shaped member provided with a recessed portion 31 having a complementary shape with the outer shapes of the tip 111 and closure member 112. Thus, the additional member 3 can be fitted on the tip 111 and the closure member 112. In other words, in case that the closure member 112 is formed into a column with a square cross-section, the recessed portion 31 is formed at its bottom and adjacent part thereof into a column with a square cross-section corresponding to that of the closure member 112 so that the closure member 112 is twisted off along with the additional member 3 when twisting the additional member 3. The additional member 3 can be fitted on the tip 111 even after twisting off the closure member 112, thus making it possible to keep the tip 111 hygienically till use after dissolving the freeze-dried medicine in the solvent to reconstruct a liquid medicine.

The barrel 11 is provided with a bypass 113 in an inner wall at the proximal end thereof, to allow the vapor to escape from the inside of the barrel to the atmosphere through the bypass 113 during freeze-drying. The gasket 12 is partially inserted into the proximal end of the barrel 11 during an operation of lyophilizing a liquid medicine filled in the barrel 11. The bypass 113 is a groove extending from the proximal end of the barrel 11 toward the distal end thereof and generally having a length shorter than a thickness of the gasket 12. The bypass 113 is configured to increase a part of the proximal end of the barrel 11 in diameter. The gasket 12 is a stopper member which is slidable along the cylindrical inner wall of the barrel 11 and generally made of an elastic material such as natural rubber, synthetic rubber (e.g., butyl-rubber, or isoprene rubber) or thermoplastic elastomer. The gasket 12 is provided at its proximal end with a cavity 121 for engagement with a head 131 of a plunger rod 13.

The solvent container 2 comprises a solvent-containing portion 21, a mouth portion 22 and a twist-off type closing member 23 mounted on the mouth portion 22, and contains a solvent charged therein. The solvent-containing portion 21 is a bag usually formed from a sheet or laminated sheet made of a chemical resistant flexible resin such as polyethylene, polypropylene, polyester or a blend thereof. Further, the mouth portion 22 and the closing member 23 are made of the same resin as that used for the solvent-containing portion 21 or an inner wall of the solvent-containing portion 21. The closing member 23 is an approximately flat, tab-shaped member except a portion for closing the mouth portion 22 and is easily removed from the mouth portion 22 by twisting it one or more times.

The mouth portion 22 is provided at its distal end 221 with an inner wall having a shape complementary to the configuration of the tip 111 of the prefilled syringe 1. Thus, the prefilled syringe 1 is liquid-tightly engaged with the solvent container 2 by inserting the tip 111 of the prefilled syringe 1 into the mouth portion 22 of the solvent container 2.

For the use of the solution kit as shown in Fig. 1, the prefilled syringe 1 and solvent container 2 are made ready by twisting off the closure member 112 and 23 from the prefilled syringe 1 and solvent container 2, respectively. Then, the prefilled syringe 1 is fluid-tightly engaged with the solvent container 2 by fitting the tip 111 of the prefilled syringe 1 in the mouth portion 22 of the solvent container 2 at the distal end 221 thereof as shown in Fig. 4. Subsequently, the plunger 13 of the prefilled syringe 1 is pulled back from the barrel 11 so that the solvent in the solvent container 2 is sucked into the barrel 11 and then, the freeze-dried medicine in the prefilled syringe 1 is dissolved in the sucked solvent. After complete dissolution of the freeze-dried medicine in the solvent, the tip 111 of the prefilled syringe 1 is removed from the mouth portion 22 of the solvent container 2. Then, the prefilled syringe is applied to injection or infusion by attachment of a needle (not shown in the drawings) to the tip 111 of the prefilled syringe.

As will be understood from the above description, the solution kit of the present invention is very easy to dissolve the freeze-dried medicine charged in the prefilled syringe, and lowers production costs because of its simple structure.

## Claims

1. A solution kit comprising:
a prefilled syringe comprising a barrel provided at a distal end thereof with a tip, a freeze-dried medicine prefilled in said barrel, and a gasket arranged in said barrel through a proximal end thereof, said tip being closed by a twist-off type closing member; and
a solvent container having a mouth portion closed by a twist-off type closing member, said container containing a solvent charged therein,
said tip of the prefilled syringe and the mouth portion of the solvent container being configured to engage with one another in liquid-tight engagement.

2. The solution kit according to claim 1, further including an additional member for twisting off the closure member, wherein the closure member on the tip of the barrel is formed into a prismatic form, and wherein the additional member is provided with a recess portion with a configuration complementary to the outer shape of the tip and closure member integrally formed therewith.
